# EUROPÄISCHE PATENTSCHRIFT

(11) **EP 1 414 693 B1**
(45) Veröffentlichungstag und Bekanntmachung des Hinweises auf die Patenterteilung: **24.01.2007**
(21) Anmeldenummer: 02791438.1
(22) Anmeldetag: 22.06.2002
(51) Int. Cl.: B62J 1/00

(54) **VERFAHREN ZUM INDIVIDUELLEN ANPASSEN VON ZWEIRADSÄTTELN**
METHOD FOR INDIVIDUALLY ADAPTING THE SADDLE OF A TWO-WHEEL VEHICLE
PROCEDE POUR L'ADAPTATION INDIVIDUELLE DE SELLES DE VEHICULES A DEUX ROUES

(30) Priorität: 25.07.2001 DE 20112013 U
(43) Veröffentlichungstag der Anmeldung: 06.05.2004
(73) Patentinhaber: Oehler, Claus, Dr., 86150 Augsburg (DE)
(72) Erfinder: Oehler, Claus, Dr., 86150 Augsburg (DE)
(74) Vertreter: Ernicke, Klaus Stefan
(86) Internationale Anmeldenummer: PCT/EP2002/006910
(87) Internationale Veröffentlichungsnummer: WO 2003/011679

(56) Entgegenhaltungen:
- DE-A- 4 324 457
- DE-A- 19 532 227
- DE-A- 19 601 974

## Beschreibung

Die Erfindung betrifft ein Verfahren zum individuellen Anpassen von Zweiradsätteln, insbesondere von Fahrradsätteln und eine Anpassvorrichtung.

Aus der DE-A-195 32 227 ist es bekannt, einen Fahrradsattel individuell an die Gesäßform des Nutzers anzupassen. Dies geschieht hier durch ein formveränderbares Sattelmaterial, z.B. durch eine flexible und mit einem formveränderlichem Material befüllbare Sattelhülle. Die Hülle kann mit Luft oder einem feinkörnigen fliesfähigen Füllmaterial befüllt sein. Die Hülle selbst besteht aus einem flexiblen Werkstoff.

Ein selbstanpassender Fahrradsattel ist auch aus der DE-A-36 25 210 bekannt. Der Sattel wird hier mit einer formbaren Masse befüllt, die zunächst flexibel und fließfähig ist und die sich dadurch selbsttätig an die Gesäßform anpasst. Das Material soll sich dann verfestigen und dadurch seine angepasste Form behalten.

Ein anderer solcher individueller Fahrradsattel ist aus der DE-A-43 27 234 bekannt. Hier soll der Sattel anatomisch individuell gegossene oder geschäumte Teile oder Einsätze verwenden.

Bei diesen vorbekannten anpassbaren Sätteln besteht das Problem, dass das verwendete Füllmaterial für die Anforderungen an einen Fahrradsattel im Dauerbetrieb nicht gut geeignet ist. Die vorbekannten angepassten Fahrradsättel können insoweit nicht alle Ansprüche befriedigen.

Aus der DE-A-43 24 457 ist eine Druckmessvorrichtung für Sitzschalen oder Rückenlehnen bekannt, bei der mittels beweglicher Messstreifen mit jeweils einem Drucksensor ein Abbild der Körperform einer Person erfasst und elektronisch gespeichert werden kann. Anhand der Messergebnisse kann ein konventioneller Sitz oder ggf. auch ein Rollstuhl für ältere Personen entsprechend angepasst werden. Die DE-A 41 31 257 zeigt eine ähnliche Vorrichtung, die mit Sensoren in Form von Kapillarschläuchen arbeitet.

Aus der DE-A 196 01 971 und der DE-A 196 01 974 sind ein Sitzprüfstempel und ein Verfahren zur quantitativen. Ermittlung des Druckkomforts eines Sitzpolsters bekannt, mit denen standardisierte Fahrzeugsitze auf ihren Druckkomfort überprüft und klassifiziert werden können. Der Sitzprüfstempel repräsentiert hierbei die Standardform eines menschlichen Körpers. Zweck dieser Technik ist es, Fahrzeugsitze herstellen zu können, die einen maximalen Komfort für möglichst viele unterschiedliche menschliche Körperformen bieten.

Die DE-A 41 31 257, DE-C 36 34 855 und DE-A 199 10 194 befassen sich mit Messanordnungen zum Messen von Kräften oder Drucken. Anhand dieser Messergebnisse kann z.B. festgestellt werden, ob sich eine Person auf einem Fahrzeugsitz befindet und welches Gewicht diese hat, um danach entsprechend einen Airbag oder andere Teile des Fahrzeugs ansteuern und insbesondere auslösen zu können.

Es ist daher Aufgabe der vorliegenden Erfindung, eine bessere Möglichkeit für eine individuelle Anpassung und Fertigung von Zweiradsätteln aufzuzeigen.

Die Erfindung löst diese Aufgabe mit den Merkmalen in den selbständigen Verfahrens- und Sachansprüchen.

Das Anpassverfahren und die dafür eingesetzte Anpassvorrichtung haben den Vorteil, dass zumindest im kritischen Bereich der Sitzfläche die lokalen Druckverhältnisse gemessen und lokalisiert werden können. Vorzugsweise wird auf der gesamten Sitzfläche gemessen.

Am Gesäß sind insbesondere die drei Auflagebereiche am Schambein und an den beiden seitlichen Sitzbeinhöckern relevant. Die örtlichen Lagen und die Druckverhältnisse dieser drei Gesäßpunkte können individuell variieren und zudem von der Sitzhaltung abhängen. Mit dem Anpassverfahren und der Anpassvorrichtung kann diesen individuellen Eigenheiten optimal Rechnung getragen werden.

Für die Anpassung wird ein entsprechender Messsattel mit einer Druckmesseinrichtung verwendet. Anhand dieser lokalisierten Druckverhältnisse kann dann der Zweiradsattel entsprechend nach Maß individuell gefertigt und an die tatsächliche Gesäßform angepasst werden. Hierfür kann er z.B. in den Problemzonen speziell gepolstert werden und/oder eine angepasste Formgebung erhalten.

Als Druckmesseinrichtung eignet sich besonders eine flexible Druckmessfolie, die sich der Sattelform optimal anpasst und die präzise Messergebnisse liefert. Der örtliche Gesäßdruck kann hierbei nach Größe und nach Lage exakt bestimmt werden. Die Auflösung der Messergebnisse ist dabei sehr fein und genau.

Um unterschiedlichen Körpergrößen und sonstigen individualisierenden Maßstäben gerecht zu werden, können mehrere standardisierte Messsättel unterschiedlicher Größe und Form benutzt werden. Hierfür können z.B. auch Unterscheidungen nach dem Einsatzzweck, z.B. nach Tourensätteln, Rennradsätteln oder dergleichen getroffen werden.

Um eine praxisgerechte Messung zu ermöglichen, besitzt die Anpassvorrichtung ein geeignetes Gestell für den Messsattel. Dieses Gestell ist ähnlich wie das jeweils betroffene Zweirad ausgestaltet und ermöglicht die Einnahme einer praxisgerechten Sitzposition bei der Messung. Das Gestell kann hierzu auch verstellbar sein, um auf unterschiedliche Fahrradgrößen, Fahrradtypen, Sitzarten etc. reagieren zu können.

In den Unteransprüchen sind weitere vorteilhafte Ausgestaltungen der Erfindung angegeben.

Die Erfindung ist in den Zeichnungen beispielsweise und schematisch dargestellt. Im einzelnen zeigen
- Figur 1:: eine Seitenansicht einer Anpassvorrichtung mit einem Messsattel und einer Druckmesseinrichtung,
- Figur 2:: eine vergrößerte Draufsicht auf den Messsattel
- Figur 3:: eine geklappte Seitenansicht des Messsattels mit der Druckmesseinrichtung und einer Auswerteeinrichtung,
- Figur 4:: eine Seitenansicht eines angepassten Zweiradsattels,
- Figur 5:: eine Draufsicht auf den angepassten Zweiradsattel von Figur 4 und
- Figur 6:: eine Seitenansicht einer Anpassvorrichtung mit einem als Halterung für Zweiräder ausgebildeten Gestell.

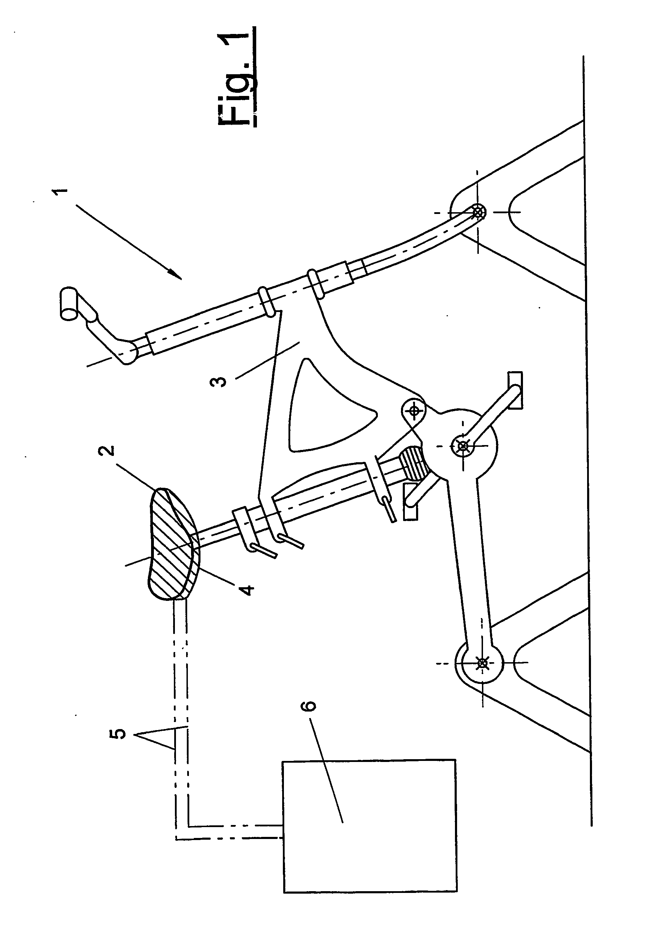

Figur 1 bis 3 und 6 zeigen eine Anpassvorrichtung (1), die die individuelle Fertigung von Zweiradsätteln (8), vorzugsweise Fahrradsätteln, nach Mass ermöglicht. Alternativ kann es sich auch um andere Sättel, insbesondere Zweiradsättel (8), z.B. Motorradsättel etc. handeln. Derartige Zweiradsättel (8) haben eine bananenähnliche oder bankartige Form, die von den Beinen des rittlings Sitzenden zumindest bereichsweise oben und seitlich umschlossen wird.

Die Anpassvorrichtung (1) besteht aus einem oder mehreren standardisierten Messsätteln (2), die eine Sattelgrundform und/oder eine Sattel-Grundgröße verkörpern. Hierbei können mehrere unterschiedliche Messsättel (2) eingesetzt werden, die sich zum einen in der Größe voneinander unterscheiden können, indem z.B. zwischen Kinder-, Damen- und Herrensätteln unterschieden wird. Außerdem können die Messsättel (2) eine unterschiedliche Form haben, die an den jeweiligen Einsatzzweck oder Fahrradtyp, z.B. Mountainbikes, Tourenräder, Rennräder etc., an unterschiedliche Komfortansprüche oder dergleichen angepasst ist. Hierbei kann es sich z.B. um einen bequemeren und für den dauerhaften bequemen und weichen Sitz geeigneten Tourensattel, einen härteren und schlankeren, bananenförmigen Rennradsattel oder dergleichen handeln.

Um eine praxisgerechte Messung zu ermöglichen, besitzt die Anpassvorrichtung (1) ein geeignetes Gestell (3) für die lösbare, austauschbare und veränderliche Anordnung des Messsattels (2). Dieses Gestell (3) kann eine beliebig geeignete Form haben. Es ist vorzugsweise ähnlich wie ein Zweirad mit Rahmen, Lenker sowie Pedalen ausgestaltet und ist auf dem Boden über Lagerböcke an Stelle der Räder ortsfest abgestützt. Es ermöglicht hierdurch dem Probanden (nicht dargestellt) die Einnahme einer praxisgerechten Sitzposition bei der Messung. Das Gestell und seine Teile können ebenfalls in der Größe, der Ausrichtung und/oder der Form verstellbar sein, um unterschiedliche Fahrradgrößen, Fahrradtypen, Lenkerstellungen, Sitzhöhen, Sitzarten etc. simulieren zu können. Einzelne Teile, z.B. der Lenker, lassen sich auch austauschen.

Durch die hochflexiblen Verstellmöglichkeiten der Anpassvorrichtung (1) können persönliche Eigenheiten des Probanden, z.B. individuelle Sitz- und Fahrhaltungen, eventuelle Körperfehlstellungen, Anomalien, Behinderungen und dgl. optimal berücksichtigt werden und gezielt in die Sattelanpassung einfließen. Desgleichen kann bei der Anpassung der vom Probanden benutzte eigene Fahrradtyp simuliert werden und Berücksichtigung finden.

Die Messsättel (2) sind vorzugsweise wenig oder gar nicht gepolstert. Die Polsterung kann dabei an allen Stellen gleichmäßig groß sein. Alternativ kann allerdings auch an bekannten Problemzonen von vornherein eine etwas stärkere Polsterung vorhanden sein.

Die Anpassvorrichtung (1) umfasst ferner eine Druckmesseinrichtung (4) zum vorzugsweise flächigen und lokalisierenden Messen des örtlichen Anpressdrucks vom Gesäß eines Probanden. Die Druckmesseinrichtung (4) ist zumindest auf oder in der Sitzfläche des Messsattels (2) angeordnet. Hierdurch ist es möglich, an einer Vielzahl von möglichst gleichmäßig auf der Sitzfläche des Messsattels (2) verteilten Punkten den lokalen Anpressdruck des Gesäßes zu messen und diesen Messwert zu lokalisieren. Insbesondere die Druckverhältnisse am Schambein und den beiden Sitzbeinhöckern können erfasst und gezielt gemessen werden.

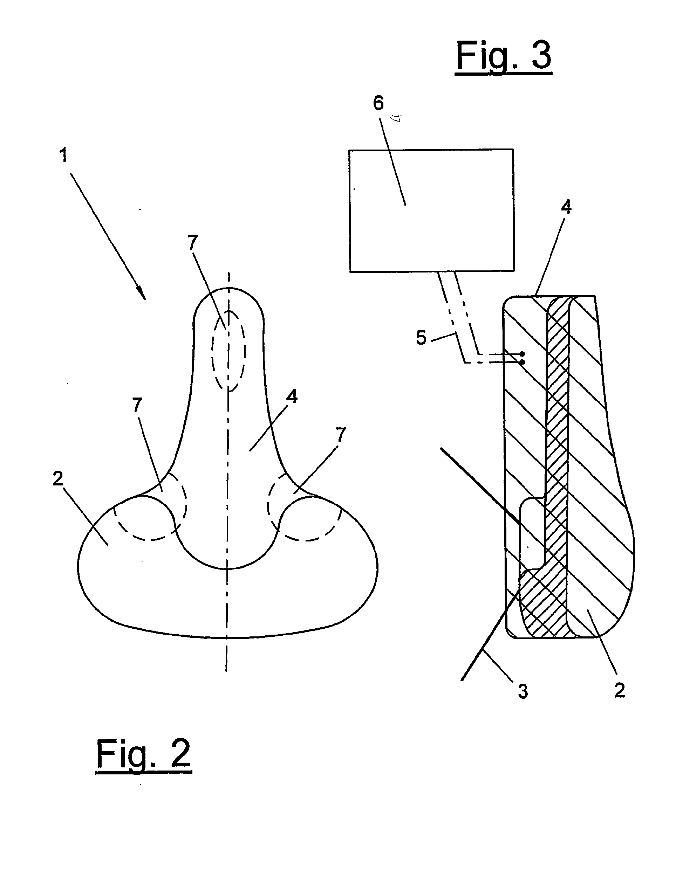

Auf diese Art kann festgestellt werden, wo ein oder mehrere Problemzonen (7) existieren, an denen das Gesäß des Probanden mit anderem, insbesondere größerem Druck als in anderen Bereichen an der Sitzfläche des Messsattels (2) anliegt. Solche Problemzonen (7) sind z.B. in Figur 2 an einem üblichen Fahrrad-Tourensattel schematisch dargestellt, welcher eine zentrale vordere Sattelspitze und ein sich daran hinten anschließendes verbreitertes Rückteil aufweist. Das Schambein liegt z.B. am Bereich der Sattelspitze auf, während die beiden seitlichen Sitzbeinhöcker an den sich verbreiternden Übergangsbereichen zum Rückteil aufliegen. Diese Problemzonen (7) sind individuell und können an völlig unterschiedlichen Stellen und mit unterschiedlicher Ausprägung und Größe auftreten. Außerdem können auch andere Zonen mit vermindertem Anpressdruck festgestellt und lokalisiert werden.

Die Druckmesseinrichtung (4) ist in der gezeigten bevorzugten Ausführungsform als hochflexible, insbesondere biegeelastische elektrische Druckverteilungsmessfolie oder -matte ausgebildet, die vorzugsweise eine hohe Zug- und Dehnfestigkeit hat und die über Leitungen (5) mit einer angeschlossenen Auswerteeinrichtung (6) verbunden ist. Die mit einem Computer oder einer anderen Recheneinheit ausgestattete Auswerteeinrichtung (6) erstellt ein Diagramm oder eine Grafik, in der die lokale Verteilung der Druckverhältnisse an der Sitzfläche des Messsattels (2) dargestellt wird. Das Diagramm bzw. die Grafik können auf einem angeschlossenen Monitor als 2D- oder ggf. auch 3D-Grafik dargestellt werden. Sie lässt sich außerdem auf jeder anderen geeigneten Ausgabeeinheit, z.B. einem Drucker, Plotter oder dgl. temporär oder dauerhaft ausgeben.

Die Druckmesseinrichtung (4) kann z.B. gemäß der DE-A-32 27 550, der DE-C-36 34 855, der DE-A-199 10 194 oder der EP-A-0 327 824 ausgebildet sein und eine Vielzahl von gleichmäßig in einer Matrix verteilten kapazitiven elektrischen Druckmesssensoren aufweisen. Alternativ kann die Druckmesseinrichtung (4) auch auf beliebige andere Art ausgebildet sein und z.B. eine Vielzahl fluidischer Sensoren gemäß der DE-41 31 257 aufweisen.

Die Druckverteilungsmessfolie (4) kann auf dem Messsattel (2) dauerhaft befestigt sein. Sie kann alternativ aber auch lose oder auswechselbar gehalten sein, z.B. in Gestalt eines an die Sattelform im Zuschnitt zumindest grob angepassten elastischen Sattelüberzugs, der sich durch seine Elastizität eng an den Messsattel (2) anschmiegt. Die Druckverteilungsmessfolie (4) kann hierbei durch Eigenelastizität, einen Spanngummi oder dgl. auf dem Messsattel (2) fixiert werden. Zudem empfiehlt es sich, über Lokalisierstifte, Fixiernasen oder -falten oder dgl. andere geeignete Passmittel eine bestimmte und reproduzierbare räumliche Zuordnung zwischen Messpunkten und Sattel zur definierten Lokalisierung der Druckmessergebnisse zu schaffen.

Zur Lokalisierung der Problemzonen (7) und der hierfür erforderlichen Kompensationsmaßnahmen, insbesondere Polsterungen (9) auf dem Messsattel (2) kann z.B. die Auswerteeinrichtung (6) einen berechneten graphischen Ausdruck der Druckverteilung am Monitor oder Drucker liefern, wobei eine 2D-Darstellung bzw. ein Druckblatt den ebenen Aufriss bzw. die Abwicklung der räumlichen Punkteverteilung wiedergibt. Der Ausdruck kann dann über entsprechende Passmarken passgenau auf den Sattel (2) gelegt werden, um hier die Ortsbereiche der erforderlichen Polsterungen (9) oder dgl. markieren zu können. Ein dem Messsattel (2) entsprechender Zweiradsattel (8) oder der Messsattel (2) selbst kann dann gemäß diesen Markierungen zur Gesäßanpassung bearbeitet werden.

In Variation kann z.B. die Lage und Ausbildung der Problemzone(n) (7) in räumlicher 3D-Darstellung auf einem Monitor, z.B. als durchsichtiges Drahtmodell, dargestellt werden. Die Darstellung wird von einem Sattler oder anderem Sattelbearbeiter optisch erfasst und auf den zu bearbeitenden Zweiradsattel (8) durch optischen Vergleich übertragen und markiert. Anhand der Markierungen kann anschließend die individuelle Sattelanpassung vorgenommen werden.

Alternativ kann die Druckmessfolie auch in den Messsattel (2) integriert sein, in dem sie die Außenhaut oder Hülle des Messsattels (2) bildet. In einer weiteren Variante kann die Druckmesseinrichtung (4) auch aus einer Vielzahl von individuellen und an bestimmten Orten dauerhaft oder temporär positionierten Druckmess-Sensoren bestehen, die allesamt über Leitungen mit einer Auswerteeinrichtung (6) verbunden sind.

Der Messsattel (2) kann eine Art mehrfach verwendbare Mess-Lehre sein, auf der die Druckmess-Sensoren in geeigneter Weise vorzugsweise dauerhaft angebracht sind. Die Form des Messsattel (2) entspricht dann der Grundform eines anzupassenden Zweiradsattels (8), auf den die Lage der gemessenen Problemzonen (7) in geeigneter Weise übertragen wird. Für unterschiedlichen Zweiradsättel (8) sind entsprechend unterschiedliche Lehren oder Messsättel (2) vorhanden. Der oder die Messsättel (2) sind hierbei Bestandteil der Anpassvorrichtung (1).

Alternativ kann der anzupassende Zweiradsattel (8) selbst den Messsattel (2) darstellen, wobei zur Druckmessung die Druckmessfolie (4) oder eine andere geeignete Sensorik temporär am Sattel angebracht wird. Die gemessenen Problemzonen (7) können am Sattel (8) in geeigneter Weise direkt markiert werden, wobei der Zweiradsattel (8) anschließend nach Entfernen der Druckmessfolie (4) bearbeitet und individuell angepasst wird. Die Druckmessfolie (4) oder dgl. ist in diesem Fall lösbar befestigt und mehrfach verwendbar. Eine flexible Druckmessfolie (4) lässt sich dabei auch für unterschiedliche Arten und Größen von Zweiradsätteln (8) einsetzen. Der Bau- und Kostenaufwand der Anpassvorrichtung (1) kann in einem solchen Fall kleiner als bei Lehren-Messsätteln sein. Zudem sind die Variationsbreite und die verfügbare Zahl der anpassbaren Sättel größer. Der Zweiradsattel (8) ist bei dieser Variante nur temporärer Bestandteil der Anpassvorrichtung (1), welche ansonsten im wesentlichen nur aus der Druckmesseinrichtung (4) und der Auswerteeinrichtung (6) sowie gegebenenfalls dem Gestell (3) besteht.

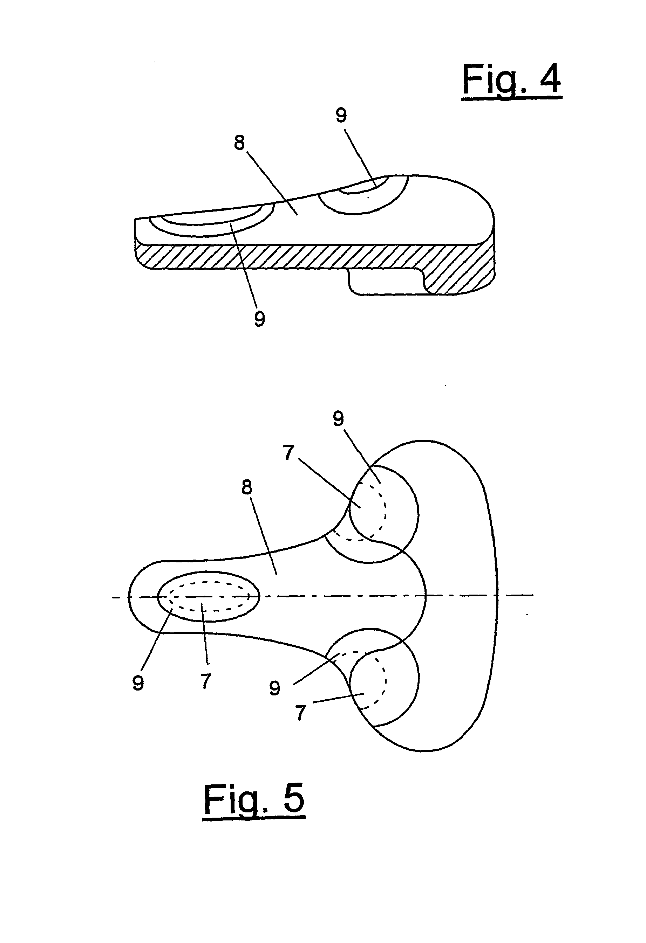

Figur 4 und 5 verdeutlichen den letztendlich nach Mass hergestellten Zweiradsattel (8), hier einen Fahrradsattel. Dieser ist entsprechend der Messergebnisse an den festgestellten Problemzonen (7) mit erhöhtem Anpressdruck und gegebenenfalls auch im umgebenden Nachbarbereich mit einer angepassten, gegebenenfalls zusätzlichen und insbesondere weicheren Polsterung (9) versehen. Die anderen Sattelbereich sind härter oder gar nicht gepolstert. Sie können hierdurch für eine gute Führung und Kraftübertragung zwischen Gesäß und Zweirad sorgen. Gegebenenfalls besteht der Zweiradsattel (8) aus einer einem Sattelgestell mit einer dünnen Sattelschale, die z.B. bei Rennsätteln relativ hart und wenig flexibel sein kann. Diese Schale ist dann nur an den Problemzonen (7) gepolstert und in den anderen Bereich relativ hart, was für die Gesäßführung vorteilhaft sein kann. Touren- oder Komfortsättel können hingegen auch außerhalb der Problemzonen (7) eine allerdings härtere Polsterung aufweisen oder auch eine erhöhte Flexibilität besitzen.

Alternativ oder zusätzlich kann die Formgebung des Zweiradsattels (8) verändert und insbesondere reduziert werden. An den Problemzonen (7) mit erhöhtem Anpressdruck wird dabei die Sattelkontur durch entsprechende Einbuchtungen oder Vertiefungen verändert und hierdurch der Anpressdruck vergleichmässigt.

An Stellen oder Zonen mit vermindertem Anpressdruck kann die Formgebung ebenfalls verändert und insbesondere durch Ausbuchtungen oder dgl. erweitert werden. Alternativ oder zusätzlich kann der Zweiradsattel (8) an diesen Bereichen verhärtet oder versteift werden, indem er durch Entfernen weicher Zonen entpolstert wird.

Der Fahrradsattel kann dadurch auch insgesamt höchst individuell und auf Mass gefertigt sein, indem er an allen Stellen der Sitzfläche exakt an die mit der Anpassvorrichtung (1) ermittelten Druckverhältnisse des individuellen Gesäßes angepasst ist und an allen Stellen der Sitzflächen eine angepasste Polsterung und/oder Formgebung besitzt. Durch die Vergleichmäßigung des Gesäß-Anpressdrucks am individuell angepassten Zweiradsattel (8) kann zugleich auch eine optimierte Formanpassung an die jeweilige Gesäßform stattfinden.

In einer weiteren nicht dargestellten Ausführungsform ist es möglich, mit einer völlig anderen Druckmesseinrichtung (4) zu arbeiten, insbesondere wenn hierbei der Zweiradsattel (8) als temporärer Messsattel (2) verwendet wird. Die Druckmesseinrichtung (4) kann in diesem Fall aus einer Bahn, Folie oder dergleichen Messlage bestehen, die über den Zweiradsattel (8) unter enger Formanpassung temporär gelegt werden kann. Die Druckmesserinrichtung (4) besteht dann aus Messelementen, die unter Druck ihre Erscheinungsform, z. B. die Farbe ändern und hierüber lokal am Messsattel (2) direkt die Problemzonen (7) mit erhöhtem Anpressdruck signalisieren. Als Messelement können hierbei druckempfindliche Siliziumschichten, aber auch druckempfindliche Gel-Schichten oder dergleichen verwendet werden. Statt einer Farbänderung kann auch eine Helligkeitsänderung, eine Oberflächenänderung oder andere geeignete auf Druck ansprechbare Anzeigen oder Signalisierungen eingesetzt werden. Bei dieser Variante können die Problemzonen (7) während der unter Umständen nur temporären Anzeige auf geeignete Weise am Zweiradsattel (8) markiert werden, z. B. durch Lochungen mit Nadeln, Farbmarkierungen oder dergleichen.

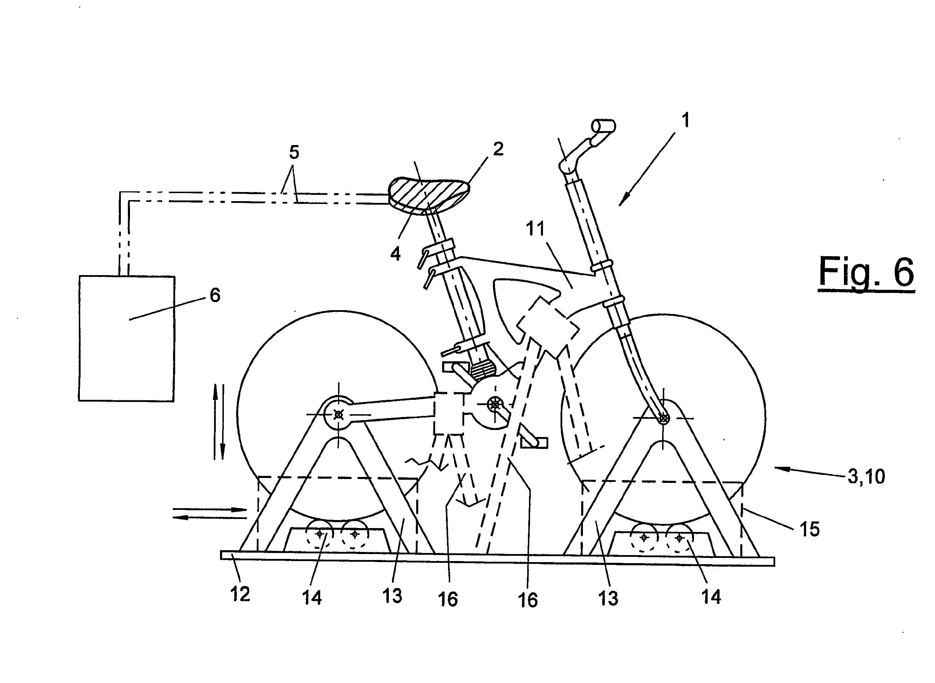

In einer in Figur 6 dargestellten Variante kann das Gestell (3) eine vorzugsweise verstellbare Halterung (10) sein, in der das eigene Zweirad (11), insbesondere Fahrrad mitsamt dem aufsitzenden Probanden zur Durchführung der Druckmessung temporär aufgenommen und fixiert werden kann. Hierdurch lässt sich eine besonders individuelle und genau an der eigenen Ausrüstung des Probanden orientierte Druckmessung und Sattelanpassung erreichen. Der Bau- und Konstruktionsaufwand für die Halterung (10) bzw. das Gestell (3) ist zudem sehr niedrig und lässt trotzdem eine vollkommene Abdeckung aller Zweiradtypen zu. Der Messsattel (2) ist hierbei vorzugsweise der eigene Zweiradsattel (8) des Probanden.

Die Halterung (10) kann z.B. aus einem Bodenteil (12) und zwei verstellbar darauf geführten Stützböcken (13) für die verschiebe- und kippsichere Aufnahme der Zweiradachsen bestehen. Die Reifen können ferner auf frei drehbaren Walzen (14) abgestützt sein, um reale Fahrsituationen zu simulieren.

Alternativ kann die Halterung (10) verstellbare Klemmschienen (15) für die Führung der Räder oder Reifen und ein oder mehrere am Zweiradrahmen lösbar und verstellbar angreifende Stützbügel (16) aufweisen. Diese Teile sind in Figur 6 gestrichelt dargestellt.

Weitere Abwandlungen der beschriebenen Erfindung sind in verschiedener Weise möglich. Beispielsweise können die konstruktive Gestaltung des Gestells (3), des Mess- und Zweiradsattels (2,8) und der Druckmess- sowie Auswerteeinrichtung (6) zur Erzielung der gleichen erfindungsgemäßen Funktion im Rahmen der Ansprüche variiert werden.

### BEZUGSZEICHENLISTE

- 1: Anpassvorrichtung
- 2: Messsattel
- 3: Gestell
- 4: Druckmesseinrichtung, Druckmessfolie
- 5: Leitung
- 6: Auswerteeinrichtung
- 7: Problemzone
- 8: Sattel, Zweiradsattel, Fahrradsattel
- 9: Polsterung
- 10: Halterung
- 11: Zweirad, Fahrrad
- 12: Bodenteil
- 13: Stützbock
- 14: Walze
- 15: Klemmeinrichtung
- 16: Stützbügel

## Patentansprüche

1. Verfahren-Zum individuellen Anpassen von Zweiradsätteln (8), insbesondere von Fahrradsätteln, **dadurch gekennzeichnet, dass** der örtliche Anpressdruck vom Gesäß eines Probanden auf einer Anpassvorrichtung (1) mit einem Messsattel (2) und einer auf oder in der Sitzfläche angeordneten Druckmesseinrichtung (4) mit einer Vielzahl von in einer Matrix verteilten Druckmesssensoren an einzelnen Stellen oder großflächig und lokalisierend gemessen und ausgewertet wird, wobei der Zweiradsattel (8) entsprechend der Messergebnisse gezielt an die individuelle Gesäßform angepasst wird.

2. Verfahren nach Anspruch 1, **dadurch gekennzeichnet, dass** der Zweiradsattel (8) an Stellen (7) mit erhöhtem Anpressdruck zusätzlich gepolstert (9) wird und/oder eine angepasste Formgebung erhält.

3. Verfahren nach Anspruch 1 oder 2, **dadurch gekennzeichnet, dass** der Zweiradsattel (8) an Stellen mit vermindertem Anpressdruck entpolstert und/oder in der Formgebung erweitert wird.

4. Verfahren nach Anspruch 1, 2 oder 3, **dadurch gekennzeichnet, dass** der Zweiradsattel (8) temporär mit einer lösbar angeordneten Druckmesseinrichtung (4) versehen und als Messsattel (2) verwendet wird.

5. Anpassvorrichtung für die individuelle Anpassung von Zweiradsätteln (8), insbesondere von Fahrradsätteln, **dadurch gekennzeichnet, dass** die Anpassvorrichtung (1) mindestens eine Druckmesseinrichtung (4) mit einer Vielzahl von in einer Matrix verteilten Druckmesssensoren und eine Auswerteeinrichtung (6) aufweist, wobei die Druckmesseinrichtung (4) auf oder in der Sitzfläche eines Messattels (2) oder Zweiradsattels (8) zum flächigen und lokalisierenden Messen des örtlichen Anpressdrucks vom Gesäßes eines Probanden dauerhaft oder temporär anbringbar ist.

6. Anpassvorrichtung nach Anspruch 5, **dadurch gekennzeichnet, dass** die Druckmesseinrichtung (4) als flexible Druckmessfolie ausgebildet ist.

7. Anpassvorrichtung nach Anspruch 5 oder 6, **dadurch gekennzeichnet, dass** die Druckmessfolie elektrische Druckmesssensoren aufweist.

8. Anpassvorrichtung nach Anspruch 5, 6 oder 7, **dadurch gekennzeichnet, dass** die Druckmesseinrichtung (4) lösbar mit dem Messsattel (2) oder dem anzupassenden Zweiradsattel (8) verbindbar ist.

9. Anpassvorrichtung nach einem der Ansprüche 5 bis 8, **dadurch gekennzeichnet, dass** die Druckmesseinrichtung (4) über Leitungen (5) an die Auswerteeinrichtung (6) anschließbar ist.

10. Anpassvorrichtung nach einem der Ansprüche 5 bis 9, **dadurch gekennzeichnet, dass** die Anpassvorrichtung (1) ein oder mehrere standardisierte Messsättel (2) unterschiedlicher Form und/oder Größe aufweist.

11. Anpassvorrichtung nach einem der Ansprüche 5 bis 10, **dadurch gekennzeichnet, dass** die Anpassvorrichtung (1) ein zweiradähnliches Gestell (3) für den Messsattel (2) oder Zweiradsattel (8) aufweist.

12. Anpassvorrichtung nach einem der Ansprüche 5 bis 11, **dadurch gekennzeichnet, dass** das Gestell (3) verstellbar ist.

13. Anpassvorrichtung nach einem der Ansprüche 5 bis 12, **dadurch gekennzeichnet, dass** das Gestell (3) als Halterung (10) für Zweiräder (11) ausgebildet ist.

## Claims

1. Method for individually adapting saddles of two-wheel vehicles (8), and in particular bicycle saddles, **characterized in that** the local pressure from the buttocks of a subject acting on an adaptation device (1) having a measuring saddle (2) and a pressure measurement device (4) arranged on or in the seat surface and having a plurality of pressure measurement sensors distributed in the form of a matrix, is measured and evaluated at individual points or generally over the surface and at specific points, the saddle of a two-wheel vehicle (8) then being adapted according to the measurement results specifically to the form of the individual buttocks.

2. Method according to Claim 1, **characterized in that** the saddle of a two-wheel vehicle (8) is provided with additional upholstery (9) and/or is provided with an adapted form at points (7) with increased pressure.

3. Method according to Claims 1 or 2, **characterized in that** the saddle of a two-wheel vehicle (8) is provided with less upholstery and/or is extended in respect of its form at points with reduced pressure.

4. Method according to Claims 1, 2 or 3, **characterized in that** the saddle of a two-wheel vehicle (8) is provided temporarily with a detachably arranged pressure measurement device (4) and is used as a measurement saddle (2).

5. Adaptation device for the individual adaptation of saddles of two-wheel vehicles (8), and in particular bicycle saddles, **characterized in that** the adaptation device (1) exhibits at least one pressure measurement device (4) having a plurality of pressure measurement sensors distributed in the form of a matrix and an evaluation device (6), the pressure measurement device (4) being capable of attachment permanently or temporarily on or in the seat surface of a measuring saddle (2) or the saddle of a two-wheel vehicle (8) for the measurement of the local pressure applied by the buttocks of a subject over the entire surface and at specific points.

6. Adaptation device according to Claim 5, **characterized in that** the pressure measurement device (4) is executed as a flexible pressure measurement film.

7. Adaptation device according to Claims 5 or 6, **characterized in that** the pressure measurement film exhibits electrical pressure measurement sensors.

8. Adaptation device according to Claims 5, 6 or 7, **characterized in that** the pressure measurement device (4) is capable of detachable attachment to the measurement saddle (2) or to the saddle of a two-wheel vehicle (8) to be adapted.

9. Adaptation device according to one of Claims 5 to 8, **characterized in that** the pressure measurement device (4) is capable of connection to the evaluation device (6) by means of wires (5).

10. Adaptation device according to one of Claims 5 to 9, **characterized in that** the adaptation device (1) exhibits one or more standardized measurement saddles (2) of different form and/or size.

11. Adaptation device according to one of Claims 5 to 10, **characterized in that** the adaptation device (1) exhibits a frame (3) resembling that of a two-wheel vehicle for the measurement saddle (2) or the saddle of a two-wheel vehicle (8).

12. Adaptation device according to one of Claims 5 to 11, **characterized in that** the frame (3) is adjustable.

13. Adaptation device according to one of Claims 5 to 12, **characterized in that** the frame (3) is executed as a support stand (10) for two-wheel vehicles (11).

## Revendications

1. Procédé pour l'adaptation individuelle de selles de véhicules à deux roues (8), en particulier de selles de bicyclettes, **caractérisé en ce que** la pression d'appui (1) locale, exercée par le fessier d'une personne-test sur un dispositif d'adaptation (1), est mesurée et exploitée soit sur certaines zones soit sur l'ensemble de la surface et de manière localisée, par une selle de mesure (2) et un dispositif de mesure de pression (4) disposé sur ou dans le siège et muni d'une pluralité de capteurs de mesure de pression répartis dans une matrice, la selle de véhicules à deux roues (8) étant spécifiquement adaptée, de manière correspondant aux résultats de mesure, à la forme individuelle du fessier.

2. Procédé selon la revendication 1, **caractérisé en ce que** la selle de véhicules à deux roues (8) est rembourré en plus (9) aux endroits (7) ayant une grande pression d'appui, et/ou reçoit une configuration adaptée.

3. Procédé selon la revendication 1 ou 2, **caractérisé en ce que** la selle de véhicules à deux roues (8) est débourrée aux endroits présentant une pression d'appui réduite et/ou reconfigurée.

4. Procédé selon la revendication 1, 2 ou 3, **caractérisé en ce que** la selle de véhicules à deux roues (8) est munie temporairement d'un dispositif de mesure de pression (4) disposé de façon amovible et est utilisé en tant que selle de mesure (2).

5. Dispositif d'adaptation pour l'adaptation individuelle de selles de véhicules à deux roues (8), en particulier de selles de bicyclettes, **caractérisé en ce que** le dispositif d'adaptation (1) a au moins un dispositif de mesure de pression (4) muni d'une pluralité de capteurs de mesure de pression répartis dans une matrice et d'un dispositif d'exploitation (6), le dispositif de mesure de pression (4) étant susceptible d'être fixé de façon permanente ou temporaire sur ou dans le siège d'une selle de mesure (2) ou d'une selle de véhicules à deux roues (8) pour effectuer des mesures, sur toute la surface et de manière localisée, de la pression d'appui locale exercée par le fessier d'une personne-test.

6. Dispositif d'adaptation selon la revendication 5, **caractérisé en ce que** le dispositif de mesure de pression (4) est réalisé en tant que feuille de mesure de pression flexible.

7. Dispositif d'adaptation selon la revendication 5 ou 6, **caractérisé en ce que** la feuille de mesure de pression a des capteurs électriques de mesure de pression.

8. Dispositif d'adaptation selon la revendication 5, 6 ou 7, **caractérisé en ce que** le dispositif de mesure de pression (4) peut être relié de façon amovible à la selle de mesure (2) ou à la selle de véhicules à deux roues (8) adaptée.

9. Dispositif d'adaptation selon l'une des revendications 5 à 8, **caractérisé en ce que** le dispositif de mesure de pression (4) peut être raccordé au dispositif d'exploitation (6) par des lignes (5).

10. Dispositif d'adaptation selon l'une des revendications 5 à 9, **caractérisé en ce que** le dispositif d'adaptation (1) a une ou plusieurs selles de mesure (2) standardisées, de formes et/ou de tailles différentes.

11. Dispositif d'adaptation selon l'une des revendications 5 à 10, **caractérisé en ce que** le dispositif d'adaptation (1) a un châssis (3) analogue à celui d'un véhicule à deux roues, pour la selle de mesure (2) ou la selle de véhicules à deux roues (8).

12. Dispositif d'adaptation selon l'une des revendications 5 à 11, **caractérisé en ce que** le châssis (3) est réglable.

13. Dispositif d'adaptation selon l'une des revendications 5 à 12, **caractérisé en ce que** le châssis (3) est réalisé en tant que fixation (10) d'un véhicule à deux roues (11).
